# EUROPEAN PATENT SPECIFICATION

(11) **EP 0 519 144 B1**
(45) Date of publication and mention of the grant of the patent: **13.08.1997**
(21) Application number: 91500066.5
(22) Date of filing: 21.06.1991
(51) Int. Cl.: A61K 9/54, A61K 31/44

(54) **New galenic process for omeprazole containing pellets**
Neues galenisches Verfahren für Omeprazol enthaltende Pellets
Nouveau procédé galenique pour des pellets contenant de l'oméprazole

(43) Date of publication of application: 23.12.1992
(73) Proprietor: ILSAN ILAC VE HAMMADDELERI SANAYI A.S., T-80300 Zincirlikuyu, Istambul (TR)
(72) Inventor: Tanberk, Ergin, Istambul (TR); Memecan, Melih, Suadiye-Istambul (TR); Güngör, Ülkü, 06700 Gaziosmanpasa, Istambul (TR); Gül, Handan, SK 1/7 Erenköy-Istambul (TR); Moralioglu, Mehmet, Istambul (TR)
(74) Representative: Alonso Langle, Emilio

(56) References cited:
- EP-A- 0 237 506
- EP-A- 0 256 933
- DE-A- 3 901 151
- GB-A- 2 189 698

## Description

A new production method for enteric coated pellets containing Omeprazole which is coated on an inert core in the form of pH buffered dispersion phase.

### Field of invention:

The present invention is related to a new production method of a stable preparation containing Omeprazole for oral administration.

### Description of invention:

Omeprazole is a potent inhibitor of gastric acid secretion. Omeprazole is a pyridine benzimidazol derivative with the following total formula C₁₇ H₁₉ N₃ O₃ S and a molecular weight of 354.4.

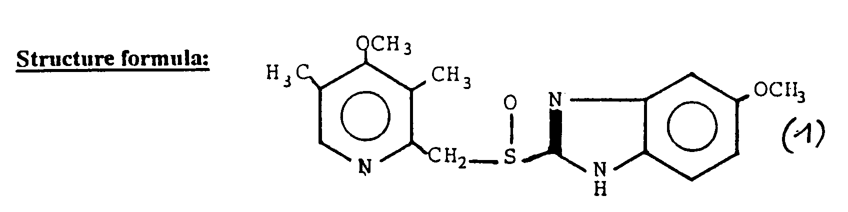

Omeprazole (1) is readily degradable in acidic enviroments, pH less than 7. Stability profile of 1 is almost the same in solid phase, and is also affected by moisture and organic solvents. The reason why oral dosage forms of Omeprazole have to be formulated as enteric coated dosage form is to protect it from acidic gastric juice (Ref.US Patent 4,786,505 Nov 22, 1988). Enteric coated pellets of Omeprazol should reasonably withstand the gastric juice but must be dissolved rapidly in the small intestine to achieve a reasonable bioavailability and of course, the effect. Several coating methods and materials have been used to comply the above mentioned prerequisites of Omeprazole (UK Patent GB 21 89 698).

In this patent application a new process for the preparation of an orally used hard gelatin capsule containing enteric coated Omeprazole pellets is described.

The invention is defined in the claims 1-3.

This new enteric coated pellet production process mainly consists of the following four steps.
I. Preparation of inert core by conventional pan coating method.
II. Active coating by using rotary type fluidized bed.
III. Protective coating by using rotary type fluidized bed.
IV. Enteric coating by using rotary type fluidized bed.

I. The contents of inert core are as following
   Saccharose **65-85 %**
   Corn Starch **15-25 %**
   Glucose **2-6 %**

   Particle size distribution range is arranged to be 90% within 0.71 mm to 0.85 mm. (in diameter) by suitable sieving. These inert pellets can also be obtained commercially.
II. To obtain a rapid dispersion active (Omeprazole) substance is micronized and sieved through 150 mesh sieves.
   The active substance sieved is dispersed in a buffered aqueous dispersion, at pH 7.1 ± 0.1, of a macromolecular binding agent. An anionic surface active agent (Sodium Lauryl Sulphate) is added to the aqueous phase to increase the wettability and smooth dispersion of Omeprazole.
   The aqueous dispersion is sprayed on to the inert pellets in the cabin of a rotary type fluidized bed machine under appropriate process parameters.
   The content of active dispersion phase for one dose (one capsule) is as following.
   Omeprazole **20 mg.**
   Hydroxypropyl methyl cellulose **5.3 mg.**
   Lactose anhydrous **8 mg.**
   L-Hydroxy propyl-cellulose **6 mg.**
   Sodium lauryl sulphate **0.5 mg.**
   Disodium hydrogen phosphate dihydrate **0.8 mg.**
   Water **0.21 ml.**
III. Active coated pellets have to be protected from the organic solvent which is normally used to disperse or dissolve the enteric coating material.
   The thickness of the layer is experimentally determined to obtain an optimal protection during the enteric coating processes and the necessary amount of coating material per capsule (one dose) for above mentioned active coated pellets (% 100 passes through 15 mesh sieves) has been determined as following.
   HPMC **3.4 mg.**
   Water **0.06 ml.**

   Aqueous molecular dispersion of HPMC is sprayed under appropriate process parameters on to the active coated pellets in the cabine of a rotary type fluidized bed machine and dried until the water content of the pellets is less than 1% when determined by the toluen distillation method described in USP XXII.
IV. Enteric coating is performed in the same machine using appropriate process parameters by spraying the following coating solution.
   HPMC phthalate **24 mg.**
   Diethyl phthalate **0.13 mg.**
   Aceton **225 mg.(... ml)**
   Ethyl alcohol **96 mg.(... ml)**

   Finished product is sieved through 15 mesh and 20 mesh sieves. Pellets which pass through 15 mesh and are retained on 20 mesh sieves, are filled into gelatin capsules. Capsule contents are 233 mg ± 10%.
II. Protective coating phase:
   Machine: Glatt GPCG 60 with GRG 30
   Active coated pellets: **25 kg ± 0.4**
   Spray nozzle: **2 x 1.8 mn**
   Nozzle position: **Tangential**
   Filter type: **PB**_{**2**} **(2% of cotton wod)**
   Sieve type: **Rotor Disc.**
   Inlet air Temperature: **50-60°C**
   Inlet Air Rate: **700-800 m**^{**3**}**/h**
   Pumping rate: **20 rpm**
   Slit width: **2 mm**
   Rotor Speed : **300 rpm**
III. Enteric coating Phase
   Machine: Glatt GPCG 60 with GRG 30
   Spray nozzle: **2 x 1.8 mm**
   Nozzle position: **Tangential**
   Filter type: **PB2**
   Sieve type: **Rotor Disc**
   Air inlet temperature: **40-50°**
   Air outlet temperature: **32-36°**
   Pumping rate: **55 rpm**
   Air inlet rate: **800 - 1000m**^{**3**}**/h**
   Rotor disk rate: **400 - 600 rpm**

## Claims

1. A production method for pellets containing Omeprazole, wherein the pellets are finally filled into a gelatine capsule, characterized in that the process consists of the following steps:
- preparation of an inert core including 65-85% of sacharose, 15-25% of starch and 2-66% of glucose, being sieved through a mesh within 0.71 and 0.85 mm,
- micronizining and sieving the active substance containing Omeprazole through a 150 mesh sieve and dispersing it in a buffered aqueous dispersion at pH = 7.1 +/- 0.1% with the addition of an anionic surface active agent,
- spraying said dispersion comprising the active substance onto the inert core pellets in the cabin of a rotary type fluidized machine,
- protective coating with HPMC in the cabin of a rotary type fluidized bed machine,
- enteric coating with HPMC phthalate, diethyl phthalate, aceton and ethyl alcohol by using a rotary type fluidized bed,
- drying to obtain a water content of less than 1 %.

2. A production method according to claim 1 in which the active substance comprises Omeprazole, hidroxy methyl cellulose, lactose anhydrous, L-hydroxy propyl-cellulose, sodium lauryl sulphate, disodium hydrogen phosphate dihydrate and water.

3. A production method according to claim 1 in which the anionic surface agent is sodium lauril sulphate.

## Patentansprüche

1. Eine Herstellungsmethode für vorgeformte Tabletten, die Omeprazol enthalten, bei der die Tabletten schliesslich in Gelatinekapseln eingebracht werden, dadurch gekennzeichnet, dass das Verfahren folgende Schritte umfasst:
- Zubereitung eines inerten Kerns, der 65-85% Saccharose, 15-25% Stärke und 2-66% Glucose enthält und durch ein Sieb mit einer Maschenweite zwischen 0,71 und 0,85 mm gesiebt wird.
- Zerkleinern des das Omeprazol enthaltenden Wirkstoffes in einer Strahlenmühle und anschliessendes Sieben durch ein Sieb mit einer Maschenweite von 150 und Dispergierung desselben in einer wässrigen Pufferdispersion mit einem pH = 7,1 +/- 0,1% unter Hinzufügen eines anionischen oberflächenaktiven Mittels.
- Sprühen dieser Dispersion, die den Wirkstoff enthält, auf die inerten Kerntabletten in der Zelle einer Wirbelbett-Drehmaschine,
- Schutzbeschichtung mit HPMC in der Zelle einer Wirbelbett-Drehmaschine.
- Entericus-Beschichtung mit HPMC Phtalat, Diethyl-Phtalat, Aceton und Ethylalkohol unter Verwendung eines Drehwirbelbettes,
- Trocknen, um den Wassergehalt auf unter 1% zu reduzieren.

2. Eine Herstellungsmethode übereinstimmend mit Anspruch 1, dadurch gekennzeichnet, dass der Wirkstoff Omeprazol, Hydroxy-methyl-cellulose, wasserfreie Lactose, L-Hydroxy-propyl-cellulose, Natrium-lauryl-sulfat, Di-Natrium-Wasserstoff-Phosphat-Dihydrat und Wasser enthält.

3. Eine Herstellungsmethode übereinstimmend mit Anspruch 1, dadurch gekennzeichnet, dass das anionische oberflächeaktive Mittel Natrium-Lauryl-Sulphat ist.

## Revendications

1. Une méthode pour produire des granulés contenant de l'Oméprazole où les granulés sont finalement introduits dans une capsule de gélatine, caractérisée en ce que le procédé est composé des étapes suivantes :
- préparation d'un noyau inerte comprenant 65-85 % de saccharose, 15-25 % d'amidon et 2-66 % de glucose, qui est tamisé à travers une maille dans les limites de 0,75 et 0,85 mm.
- micronisation et tamisage de la substance active qui contient de l'Omeprazole à travers un tamis de 150 de maille et sa dispersion dans une dispersion tampon aqueuse à pH = 7,1 +/- 0,1 % en ajoutant un agent tensoactif anionique,
- pulvérisation de cette dispersion qui contient la substance active sur les granulés du noyau inerte dans la chambre d'une machine à lit fluidifié du type rotatif,
- revêtement de protection en HPMC dans la chambre d'une machine à lit fluidifié du type rotatif,
- revêtement entérique avec du phtalate HPMC, du phtalate de diéthyle, de l'acétone et de l'alcool éthylique en utilisant un lit fluidifié du type rotatif,
- séchage pour obtenir une teneur en eau inférieure à 1 %.

2. Une méthode de production selon la Revendication 1 dans laquelle la substance active contient de l'Omeprazole, de la cellulose de méthyle hydroxylé, de l'anhydre de lactose, de la cellulose propylique hydroxylé-L, du sulfate de lauryle de sodium, du dihydrate de phosphate d'hydrogène de disodium et de l'eau.

3. Une méthode de production selon la Revendication 1 dans laquelle l'agent de surface anionique est du sulfate de lauryle de sodium.
